# EUROPÄISCHE PATENTSCHRIFT

(11) **EP 2 062 965 B1**
(45) Veröffentlichungstag und Bekanntmachung des Hinweises auf die Patenterteilung: **12.03.2014**
(21) Anmeldenummer: 08166351.0
(22) Anmeldetag: 10.10.2008
(51) Int. Cl.: C12M 1/107

(54) **Behälterabdeckung mit Gasmembran**
Container cover with gas membrane
Couvercle de récipient doté d'une membrane de gaz

(30) Priorität: 26.11.2007 DE 102007057118
(43) Veröffentlichungstag der Anmeldung: 27.05.2009
(73) Patentinhaber: AGROTEL GmbH, 94152 Neuhaus am Inn (DE)
(72) Erfinder: Laner, Cyriak, 94152 Neuhaus am Inn (DE)
(74) Vertreter: Benninger, Johannes

(56) Entgegenhaltungen:
- EP-A- 1 801 037
- WO-A-83/03884
- DE-A1-102006 005 859
- DE-C- 40 779

## Beschreibung

Die vorliegende Erfindung betrifft eine Vorrichtung zur gasdichten Abdeckung großer Behälter, insbesondere von Fermentern, Nachgärlagern, Endlagern und Biogasanlagen, mit den Merkmalen des Oberbegriffs des Anspruchs 1 sowie ein Verfahren zur Variierung des Innenvolumens in Fermentern, Nachgärlagern, Endlagern und Biogasanlagen mit den Merkmalen des Oberbegriffs des Anspruchs 7.

### Stand der Technik

Im Bereich der Biogasanlagen / Fermentationsanlagen etc. wird vielfach in geschlossenen Systemen gearbeitet. Das bedeutet, dass die Systeme nach allen Seiten hin fluiddicht abgeschlossen werden müssen, damit alle Komponenten im System verbleiben und nur kontrolliert entfernt werden können.

Hierbei muss weiterhin beachtet werden, dass aufgrund der Entwicklung von Biogas innerhalb dieser Anlagen das benötigte Raumvolumen unter Umständen schwankt, so dass eine gewisse Flexibilität wenigstens einer Wand, beispielsweise der oberen Abdeckung, von Vorteil ist.

EP 1 200 551 B1 zeigt eine Biogasfermenteranlage mit einer dachförmigen Schutzabdeckung und mit einer mittig angeordneten Tragsäule. DE 103 54 598 B4 beschreibt ebenfalls eine mittige Abstützung des Behälters. DE 20 2006 014 149 U1 zeigt ein mittiges Stützelement, das auf einem Fundament abgestützt ist. Das Stützelement dient zur Abspannung der Behälterdecke

WO 8303884 A zeigt einen Biogasreaktor mit einer flexiblen Abdeckung für einen Gasspeicher mit einem flexiblen Nutzvolumen bestehend aus einer gasdichten Folienkonstruktion. Dabei ist das obere Ende eines Rohres gasdicht mit der Folie verbunden. Beim Heben und Senken der Abdeckung bewegt sich das Rohr, wobei es gleitend einer Stange geführt ist. Die Bewegung der inneren Membran ist durch einen Rost nach unten begrenzt.

EP 1801037 A zeigt eine Abdeckung für einen nach oben offenen Biogasspeicher mit einem Schwimmkörper, der der Abdeckung die gewünschte Form und Eigensteifigkeit verleiht. Als bekannten Stand der Technik beschreibt EP 1801037 A einen beispielsweise zylindrischen Güllebehälter mit einer zentralen Stütze und einer ersten Wetterschutzmembran.

DE 102006005859 A beschreibt einen Biogastank, in dem das entstehende Biogas unter einer Speicherfolie gesammelt wird.

Weiterhin sind Tragluftdachkonstruktionen mit integriertem Gasspeicher bekannt, bei denen die Außenmembran bzw. das Tragluftdach mit einem Radialgebläse auf dem notwendigen Druck gehalten wird, um den klimatischen Anforderungen (Regen, Schnee und Wind) zu entsprechen. Die Innenmembran ist am Behälterrand mit der Außenmembran gasdicht angebracht. Sie ist kleiner als die Außenmembran konfektioniert, damit zwischen den beiden immer ein bestimmes Luftpolster gewährleistet wird, dieses garantiert einerseits das notwendige Tragluftpolster und stellt andererseits eine zusätzliche Isolierung dar.

Die genannten Abdeckungen erlauben eine gewisse Flexibilität, allerdings muss bei der Produktion einer größeren Menge an Biogas dieses relativ schnell aus dem System entfernt werden, da das Innenvolumen des Bioreaktors begrenzt ist.

### Beschreibung

Das Ziel der Erfindung besteht darin eine fluiddichte Behälterabdeckung aus einer äußeren und einer inneren Membran zu konstruieren, die einfach dem internen Volumenbedarf angepasst werden kann, wobei die innere Membran wartungsfrei und fluiddicht geführt ist.

Dieses Ziel der Erfindung wird mit dem Gegenstand des unabhängigen Anspruchs erreicht. Merkmale vorteilhafter Weiterbildungen der Erfindung ergeben sich aus den abhängigen Ansprüchen.

Die Erfindung betrifft eine fluiddichte Abdeckung für einen Biogasreaktor. Die fluiddichte Abdeckung umfasst eine äußere und eine innere Membran bzw. Schutzfolie und einem dazwischen liegenden Hohlraum. Die äußere Membran wird über das obere Ende einer Mittelstütze in Form gehalten und die Innenmembran wird an der Mittelstütze geführt. Vorzugsweise ist die Innenmembran fluiddicht mit einem beweglichen, flexiblen Schlauch verbunden, wobei der Schlauch die Mittenstütze fluiddicht umfasst und entlang derselben beweglich ist.

Bei einer Biogasanlage mit erfindungsgemäßer Behälterabdeckung kann es sich beispielsweise um eine Vorrichtung mit kreisförmigem Grundriss handeln. Der Fermenter bzw. Biogasreaktor besteht aus einer flüssigkeitsdichten Betonfußplatte. Die Form dieser Betonfußplatte kann dabei unterschiedlich gestaltet sein. beispielsweise kreisförmig oder rechteckige Ausführungsformen etc.

Auf dieser Fußplatte befinden sich die senkrecht dazu angeordneten, vertikalen Seitenwände. Diese bestehen beispielsweise ebenfalls aus Beton. Vorzugsweise werden die Fußplatte und die Seitenwände gleichzeitig gegossen, so dass eine fluiddichte Verbindung zwischen diesen entsteht.

Auf die Seitenwände setzt die Dachkonstruktion auf. Diese kann eine Dachneigung von 20° bis 30° aufweisen. Gemäß einem bevorzugten Ausführungsbeispiel besteht die Dachabdeckung aus einer Wetterschutzplane, die mittig über einen gewölbten Boden geführt ist.

Gemäß einer weiteren Ausführungsform kann die Dachkonstruktion auch aus einem gegossenen Betonteil bestehen, dass fluiddicht mit den Seitenteilen verbunden wird.

Im mittleren Bereich des Fermenters ist eine Stütze, beispielsweise aus Edelstahl, angeordnet, die am oberen Ende durch den Klöpperboden zur Führung der Wetterschutzfolie begrenzt wird. Die Stütze kann eine Fußplatte aufweisen, mit der Sie auf die Bodenplatte der Biogasanlage aufsetzt und / oder fest verbunden ist. Die Bodenplatte des Fermenters kann in dem Bereich, in dem die Mittelstütze aufsetzt verstärkt sein.

Gemäß einer weiteren Ausführungsform sind in die Wetterschutzplane Gurte integriert / einkonfektioniert. Am oberen Ende der mittigen Stütze befindet sich bei dieser Ausführungsform ein so genannter Gurtboden mit einem Kopf für die Gurtaufnahme. An der Behälterwand befinden sich Ratschen, mit denen die Gurte der Außenmembran entsprechend gespannt werden können.

Gemäß einer bevorzugten Ausführungsform besteht die verwendete Wetterschutzplane beispielsweise aus Polyestergewebe mit beidseitiger PVC Beschichtung und ist kegelförmig konfektioniert. Außen herum ist ein sogenannter Hohlsaum angeordnet, in dem sich das Abspannrohr / Formrohr befindet. Im Abstand von beispielsweise 1,00 m können Edelstahl Spannratschen an der Behälterwand und am Abspannrohr befestigt, welche die gesamte Plane straff spannen. Oben auf der mittig angeordneten Stütze ist ein Edelstahlpilz bzw. Köppelboden aufgesetzt, an dem die Abdeckung befestigt wird.

Unterhalb der Dachkonstruktion befindet sich innerhalb des Biogasreaktors eine zusätzlich Abdeckung. Dabei handelt es sich um eine bewegliche Innenmembran, die zum einen im oberen Bereich der Seitenwände, insbesondere an der Krone der Seitenwände gasdicht an diesen befestigt ist und zum anderen fluiddicht an einen beweglichen Schlauch anschließt. Der bewegliche Schlauch umfasst weiterhin fluiddicht die mittige Stütze im oberen Bereich und ist entlang der Stütze beweglich. Das flexible Schlauchteil wird über die Stütze geführt, wodurch sich die daran befestigte Innenmembran heben und senken kann.

Bedingt durch die variable Positionierung der Innenmembran ändert sich die Größe des Hohlraums zwischen der Innen- und der Außenmembran.

Steigt die Menge an Biogas innerhalb der Anlage, so wird der Schlauch entlang der Stütze nach oben geschoben. Entsprechend bewegt sich die an den Schlauch gekoppelte Innenmembran nach oben, so dass sich das innere Volumen im Reaktor erhöht. Bei Entnahme von Biogas reguliert sich das System entsprechend selbst und die Innenmembran sinkt wieder ab.

Die Bewegung des Schlauchs entlang der Mittelstütze wird durch eine Blockiervorrichtung an der Mittelstütze nach unten begrenzt. Dadurch wird die Bewegung der Innenmembran ebenfalls nach unten begrenzt und es wird verhindert, dass der Schlauch und somit die daran gekoppelte Innenmembran zu weit nach unten absinkt.

Die Bewegung des Schlauchs nach oben wird durch die Größe der Innenmembran und der Länge des flexiblen Schlauches begrenzt. Befindet sich die Innenmembran im vollständig nach oben gespannten Zustand, so ist ein weiteres nach oben rutschen des Schlauchs unmöglich.

Durch diese bewegliche Innenmembran entsteht ein sich selbst regulierendes System, bei dem das Innenvolumen der Biogasanlage entsprechend der Biogasmenge angepasst ist, und zwar dadurch, dass sich die Innenmembran entsprechend dem Biogasvolumen nach oben oder nach unten bewegt.

### Figurenbeschreibung

Weitere Merkmale, Ziele und Vorteile der vorliegenden Erfindung gehen aus der nun folgenden detaillierten Beschreibung einer bevorzugten Ausführungsform der Erfindung hervor, die als nicht einschränkendes Beispiel dient und auf die beigefügten Zeichnungen Bezug nimmt. Gleiche Bauteile weisen dabei grundsätzlich gleiche Bezugszeichen auf und werden teilweise nicht inehrfach erläutert.
- Fig. 1: zeigt eine erfindungsgemäße doppelte Behälterabdeckung mit variablem Gasspeichervolumen;
- Fig. 2: zeigt die fluiddichte Verbindung zwischen den Seitenwänden und der Behälterabdeckung und
- Fig. 3: zeigt die fluiddichte Verbindung zwischen den Seitenwänden und der Behälterabdeckung bei der Verwendung von Gurten.

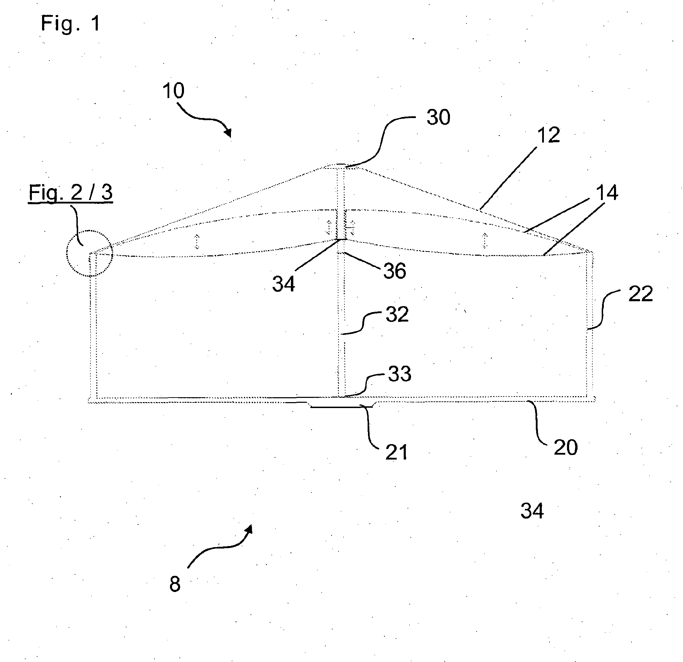

Eine mögliche Ausgestaltung einer Abdeckung 10 einer Biogasanlage 8 wird anhand von Figur 1 illustriert.

Der Biogasreaktor 8 weist eine flüssigkeitsdichte Betonfußplatte 21 und darauf angeordnete, Seitenwände 22 auf. Diese bestehen beispielsweise ebenfalls aus Beton und werden vorzugsweise gleichzeitig mit der Fußplatte 21 gegossen, so dass eine fluiddichte Verbindung zwischen der Grundplatte 21 und den Seitenwänden 22 entsteht.

Auf die Seitenwände 22 setzt die Dachkonstruktion 10 auf. Diese besteht aus einer äußeren und einer inneren Membran 12, 14. Die äußere Membran 12 weist eine Dachneigung von 20° bis 24° auf und besteht aus einer Wetterschutzfolie, die mittig über einen gewölbten Boden (Klöpperboden) 30 geführt ist.

In der Mitte des Biogasreaktors 8 befindet sich eine Edelstahlstütze 32. Diese ist an ihrem unteren Ende in der Bodenplatte 21 verankert, die in diesem Bereich eine Verstärkung aufweisen kann. Am oberen Ende der Stütze 32 ist ein Stützenpilz oder Klöpperboden 30 angeordnet, über den die äußere Membran 12 geführt ist.

Die Mittelstütze 32 kann beispielsweise einen runden Querschnitt aufweisen, es ist jedoch auch die Verwendung von Edelstahlträgem mit einem anderen Profil möglich. Die Mittelstütze 32 kann auch aus einem anderen Material bestehen. Wichtig ist hierbei, dass das gewählte Material korrosionsbeständig ist, da es in einem Biogasreaktor diversen chemischen Substanzen, beispielsweise Schwefelgasen etc., augesetzt ist.

Weiterhin weist die Mittelstütze eine verbreiterte Bodenplatte 33 auf, die mit der Stütze fest verbunden, beispielsweise verschweißt ist. Diese Bodenplatte 33 steht auf der verstärkten Bodenplatte 21 und kann mit dieser ebenfalls fest verbunden, beispielsweise verschraubt sein.

Unterhalb der äußeren Dachmembran 12 befindet sich innerhalb der Biogasanlage 8 eine zusätzlich Abdeckung 14. Dabei handelt es sich um eine Innenmembran 14, die zum einen im oberen Bereich der Seitenwände 22 gasdicht an diesen befestigt ist und zum anderen fluiddicht an einen beweglichen Schlauch 34 anschließt, der die mittige Stütze 32 im oberen Bereich umfasst.

Die Mittelstütze 32 weist in ihrem oberen Bereich eine Blockiervorrichtung 36 auf, die verhindert, die die Bewegung des beweglichen Schlauchs 34 und der angekoppelten Innenmembran 14 nach unten hin begrenzt.

Die Blockiervorrichtung 36 kann beispielsweise ein in einer bestimmten Höhe an die Stütze 32 angeschweißter Metallring, insbesondere ein Edelstahlring sein. Weist die Stütze keinen runden Querschnitt auf, ist die Blockiervorrichtung entsprechend dem vorliegenden Querschnitt angepasst. Weiterhin ist es möglich, dass als Blockiervorrichtung 36 ein eng anliegender, unverrutschbarer Gummiring oder ähnliches Verwendung findet. Die Stütze 32 kann beispielsweise eine entsprechende Einkerbung aufweisen, an die der Gummiring fast anliegt und nicht mehr seine Position ändern kann.

Das flexible Schlauchteil 34 wird über die Stütze 32 geführt, wodurch sich die daran befestigte Membran heben und senken kann. Die Bewegung wird nach unten hin durch die Blockiervorrichtung 36 und nach oben hin durch die Größe der Innenmembran 14 und durch die Länge des flexiblen Schlauches begrenzt.

Steigt die Menge an Biogas innerhalb der Anlage 8, so wird der Schlauch 34 entlang der Stütze 32 nach oben geschoben. Entsprechend bewegt sich die an den Schlauch 34 gekoppelte Innenmembran 12 nach oben, so dass sich das innere Volumen im Reaktor 8 erhöht. Bei Entnahme von Biogas reguliert sich das System entsprechend selbst und die Innenmembran 14 sinkt wieder ab.

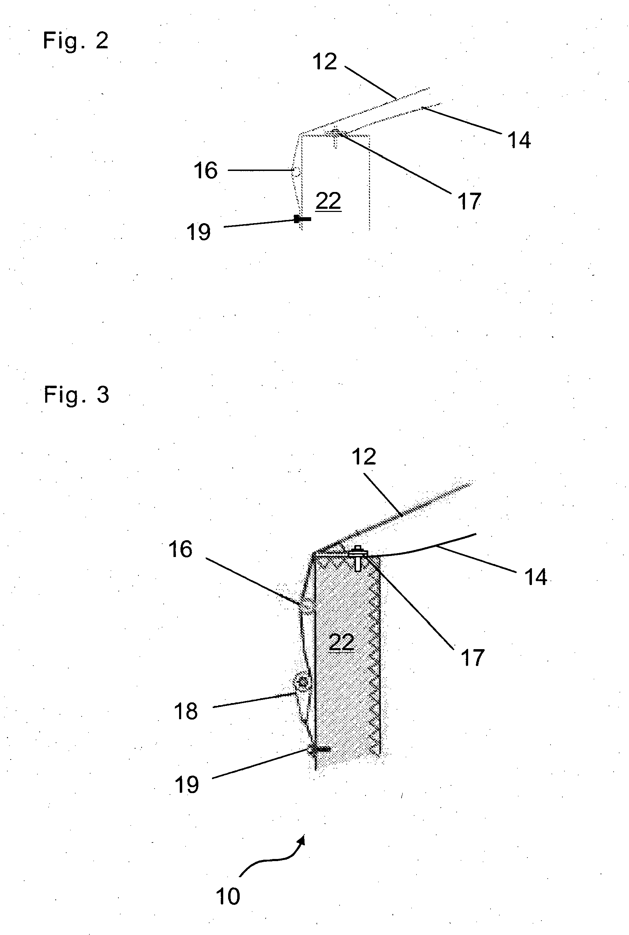

Fig. 2 und 3 zeigen zwei unterschiedliche Möglichkeiten zur fluiddichten Verbindung zwischen den Seitenwänden 22 der Biogasanlage 8 und der aus einer inneren und einer äußeren Membran 14, 12 bestehenden Dachkonstruktion 10.

In Fig. 2 wird die äußere Membran 12 mittels eines Formrohrs 16 über die Seitenwand 22 geführt und gestrafft und anschließend mit einem Befestigungselement, beispielsweise mittels einer Schraube 19, fest mit der Außenseite der Seitenwand 22 verbunden. Die innere Membran 14 wir dagegen mittels einer Befestigungsvorrichtung 17 auf der Krone der Seitenwand 22 befestigt. Die Befestigungsvorrichtung 17 kann beispielsweise aus mehreren Stahlprofilen und / oder Zellkautschukdichtungen bestehen, die die Innenmembran fluiddicht einklemmen und mit der Seitenwand 22 fest verbunden, insbesondere verschraubt sind, d.h. die Befestigung erfolgt mittels einer gasdichten Verschraubung der Innenmembran.

Fig. 3 stellt die Situation dar, wenn die äußere Membran 12 zusätzlich mit Hilfe von in der Membran 12 integrierten Gurten gespannt werden kann. Die innere Membran 14 ist wiederum mittels einer Befestigungsvorrichtung 17 auf der Krone der Seitenwand 22 befestigt (vgl. Figurenbeschreibung zu Fig. 2). Die äußere Membran 12 wird mittels eines Formrohrs 16 über die Seitenwand 22 geführt und mittels einer Spannratsche 18 gestrafft. Unterhalb der Spannratsche 18 ist die äußere Membran 12 mit einem Befestigungselement, beispielsweise mittels einer Schraube 19, fest mit der Außenseite der Seitenwand 22 verbunden.

### Bezugszeichenliste

- 8: Biogasreaktor
- 10: Dachkonstruktion
- 12: äußere Membran
- 14: innere bewegliche Membran
- 16: Formrohr
- 17: Befestigungsvorrichtung
- 18: Ratsche
- 19: Schraube
- 21: Bodenplatte (mit Fundamentverstärkung)
- 22: Seitenwände
- 30: Stützpilz oder Klöpperboden
- 32: Mittelstütze
- 33: Stützenfuß
- 34: beweglicher Schlauch
- 36: Blockiervorrichtung / Begrenzungsvorrichtung

## Patentansprüche

1. Fluiddichte Abdeckung (10) für einen Biogasreaktor (8) bestehend aus einer äußeren (12) und einer inneren Membran (14) bzw. Schutzfolie und einem dazwischen liegenden Hohlraum, wobei die äußere Membran (12) über das obere Ende einer Mittelstütze (32) in Form gehalten ist und die Innenmembran (14) an der Mittelstütze (32) geführt ist, **dadurch gekennzeichnet, dass** die Innenmembran (14) fluiddicht mit einem beweglichen, flexiblen Schlauch (34) verbunden ist, wobei der Schlauch (34) die Mittelstütze (32) fluiddicht umfasst und wobei der Schlauch (34) entlang der Mittelstütze (32) beweglich ist.

2. Fluiddichte Abdeckung gemäß Anspruch 1, **dadurch gekennzeichnet, dass** die Bewegung des Schlauchs (34) nach unten begrenzt ist (36).

3. Fluiddichte Abdeckung gemäß einem der voranstehenden Ansprüche, **dadurch gekennzeichnet, dass** die Bewegung des Schlauchs (34) nach oben hin durch die Größe der Innenmembran (14) und den Weg des flexiblen Schlauches (34) begrenzt ist.

4. Fluiddichte Abdeckung gemäß einem der voranstehenden Ansprüche, **dadurch gekennzeichnet, dass** die äußere Membran (12) eine Wetterschutzplane mit integrierten Gurten ist.

5. Fluiddichte Abdeckung gemäß Anspruch 1, **dadurch gekennzeichnet, dass** die Behälterwand (22) Ratschen (18) zum Spannen der Gurte der äußeren Membran (12) aufweist.

6. Fluiddichte Abdeckung gemäß einem der Ansprüche 1 oder 2, **dadurch gekennzeichnet, dass** die Fußplatte (20) und die Seitenwände (22) fluiddicht verbunden sind.

7. Verfahren zur Regulierung des Innenvolumens einer Biogasanlage (8) entsprechend der Menge an produziertem Biogas, **dadurch gekennzeichnet, dass** sich das Innenvolumen einer Biogasanlage gemäß einem der Ansprüche 1 bis 6 selbst reguliert.

8. Verfahren gemäß Anspruch 9, **dadurch gekennzeichnet, dass** sich die innere Membran (14) entsprechend dem Biogasvolumen nach oben oder nach unten bewegt.

## Claims

1. Fluid- tight cover (10) for a biogas reactor (8) comprising an outer membrane (12) and an inner membrane (14) or protective foil and an intermediate hollow space, wherein the outer membrane (12) is held in form over the upper end of a central support (32) and wherein the inner membrane (14) is guided on the central support (32), **characterized in that** the inner membrane (14) is connected in a fluid- tight manner to a movable flexible tube (34), wherein the tube (34) envelops the central support (32) a fluid- tight manner and wherein the tube (34) is movable along said central support (32).

2. Fluid- tight cover according to claim 1, **characterized in that** the downward movement of the tube (34) is limited (36).

3. Fluid- tight cover according to one of the previous claims, **characterized in that** the upward movement of the tube (34) is limited by the size of the inner membrane (14) and the path of the flexible tube (34).

4. Fluid- tight cover according to one of the previous claims, **characterized in that** the outer membrane (12) is an awning with integrated belts.

5. Fluid- tight cover according to claim 1, **characterized in that** the reactor wall (22) comprises ratchets (18) for tensioning the belts of the outer membrane (12).

6. Fluid- tight cover according to claims 1 or 2, **characterized in that** the bottom plate (20) and the sidewalls (22) are connected in a fluid- tight manner.

7. Method for regulating the internal volume of a biogas reactor (8) according to the amount of biogas produced, **characterized in that** the internal volume of a biogas reactor regulates itself according to one of claims 1 to 6.

8. Method according to claim 9, **characterized in that** the inner membrane (14) moves upwards or downwards corresponding to the biogas volume.

## Revendications

1. Couverture (10) étanche aux fluides pour un réacteur à biogaz (8), se composant d'une membrane extérieure (12) et d'une membrane intérieure (14) ou bien feuille de protection ainsi que d'un vide situé entre celles-ci, ladite membrane extérieure (12) étant maintenue en forme par l'extrémité supérieure d'un étai central (32) et ladite membrane intérieure (14) étant guidée sur ledit étai central (32), **caractérisée par le fait que** ladite membrane intérieure (14) est reliée de manière étanche aux fluides à un tuyau souple (34) déplaçable flexible, ledit tuyau souple (34) entourant ledit étai central (32) de manière étanche aux fluides et ledit tuyau souple (34) étant déplaçable le long dudit étai central (32).

2. Couverture étanche aux fluides selon la revendication 1, **caractérisée par le fait que** le mouvement du tuyau souple (34) est limité vers le bas (36).

3. Couverture étanche aux fluides selon l'une quelconque des revendications précédentes, **caractérisée par le fait que** le mouvement du tuyau souple (34) est limité vers le haut par la taille de ladite membrane intérieure (14) et par la course du tuyau souple (34) flexible.

4. Couverture étanche aux fluides selon l'une quelconque des revendications précédentes, **caractérisée par le fait que** ladite membrane extérieure (12) est une bâche de protection contre les intempéries ayant des sangles intégrées.

5. Couverture étanche aux fluides selon la revendication 1, **caractérisée par le fait que** la paroi de récipient (22) présente des arrêts par cliquet (18) destinés à tendre lesdites sangles de la membrane extérieure (12).

6. Couverture étanche aux fluides selon l'une quelconque des revendications 1 ou 2, **caractérisée par le fait que** la plaque de base (20) et les parois latérales (22) sont reliées entre elles de manière étanche aux fluides.

7. Procédé de régulation du volume intérieur d'une installation à biogaz (8) selon la quantité de biogaz produit, **caractérisé par le fait que** le volume intérieur d'une installation à biogaz est auto-régulateur selon l'une quelconque des revendications 1 à 6.

8. Procédé selon la revendication 9, **caractérisé par le fait que** ladite membrane intérieure (14) se déplace vers le haut ou vers le bas en fonction du volume de biogaz.
